# EUROPEAN PATENT APPLICATION

(11) **EP 1 676 901 A1**
(43) Date of publication of application: **05.07.2006**
(21) Application number: 05258007.3
(22) Date of filing: 22.12.2005
(51) Int. Cl.: C10G 11/22, C07C 5/48

(54) **Refrigeration system for the production and recovery of olefins**

(30) Priority: 30.12.2004 US 26212
(71) Applicant: Innovene USA LLC, Chicago, IL 60601 (US)
(72) Inventor: Reyneke, Rian, Kay, TX 77450 (US); Foral, Michael J., Aurora, IL 60504 (US); Amelse, Jeffrey A., Batavia, IL 60504 (US)
(74) Representative: King, Alex

(57) **Abstract**

An autothermal cracking process for production and recovery of olefins. The process comprises feeding a substantially hydrocarbon feedstock and an oxygen-containing g as to an a utothermal cracker to provide a hydrocarbon product stream comprising olefins. A waste enthalpy source generated by said autothermal cracking process is used to at least partially drive an ammonia absorption refrigeration system to provide chilling for at least one process stream in the separation and/or purification of olefins from the hydrocarbon product stream.

In addition, an ammonia absorption refrigeration process comprising at least one enthalpy source selected from the group consisting of: quench water generated through the cooling of cracked gases from an autothermal cracking reactor; steam generated through the cooling of cracked gases from an autothermal cracking reactor; sufficiently warm streams derived from processes which utilize the ethylene produced from the autothermal cracking process; and sufficiently warm streams from other chemical or refinery process units located near an autothermal cracking reactor.

## Description

### Background of the Invention

Autothermal cracking is a process for the manufacture of olefins in which a hydrocarbon feed is mixed with oxygen and passed over an autothermal cracking catalyst. The autothermal cracking catalyst is capable of supporting combustion beyond the fuel rich limit of flammability. Combustion is initiated on the catalyst surface, and the heat required to raise the reactants to the process temperature and to carry out the endothermic cracking process is generated in situ. Generally, the hydrocarbon feed and the oxygen is passed over a supported catalyst to produce the olefin product. The autothermal cracking process is described in EP 332289B; EP-529793B; EP-A-0709446 and WO 00/14035.

As with conventional furnace-based cracking, the product stream exiting the autothermal reactor is typically quenched by contact with water, and subsequently passed through a series of separation and purification steps. The product stream usually undergoes separation and purification steps to remove hydrogen, methane and CO₂. The reaction products are then treated to separate methane, hydrogen and carbon monoxide before the remaining product stream is treated in order to separate a C₂ containing stream from heavier hydrocarbons. The C₂ containing stream is treated to separate ethylene from ethane. The remaining product stream, comprising C₃ and higher hydrocarbons, may be further treated to separate propane and propylene, for example.

Ammonia absorption refrigeration (hereinafter referred to as "AAR") systems differ from compressor-based refrigeration systems (such as a conventional C₃ refrigeration system) in that they require only a relatively low-level source of enthalpy, rather than high-level energy. For example, AAR systems can be driven by energy available at temperatures as low as 95°C, while compressor-based refrigeration systems typically require either superheated high-pressure steam or electricity to drive the compressors. This enthalpy is generally a waste heat source that would otherwise be lost to the atmosphere. AAR systems can therefore be more energy efficient than C₃ refrigeration systems. AAR can be a cost-effective, energy saving process that can be used for providing moderate temperature refrigeration.

In a relatively simple AAR system, an enthalpy source such as waste heat reboils an ammonia fractionator that is fed a rich ammonia aqua stream comprising a relatively high concentration of ammonia in water. The fractionator separates the strong aqua stream into a higher purity ammonia vapor overhead stream, and a bottoms stream with a lower concentration of ammonia relative to the strong aqua stream. The ammonia vapor overhead stream is condensed, typically via air or water cooling, to generate liquid ammonia refrigerant. The liquid ammonia refrigerant is then directed to the refrigerant users. As enthalpy is transferred indirectly from the material being refrigerated, the liquid ammonia refrigerant evaporates and generates ammonia refrigerant vapor. The ammonia vapor is directed to an absorber, a long with the bottoms stream from the fractionator which absorbs the ammonia vapor while releasing heat of absorption. The heat of absorption is typically removed by water cooling the absorber. Various, attempts to use AAR systems to replace the propane or propylene refrigeration system in olefins manufacture have generally not been successful. There are two basic problems with using AAR systems in conventional olefins plants. First, the quench water heat available in a conventional olefins plant is not available at a high enough temperature to drive the AAR system. Second, conventional furnaces produce a relatively large amount of high-pressure steam by recovering h igh-temperature energy f rom the furnace flue gases. Conventional C₃ refrigeration systems consume a significant fraction of this high-pressure steam, thereby helping achieve a steam balance in the olefins plant. A conventional olefins plant that utilizes an AAR system, rather than a C₃ refrigeration system, would likely be significantly out of steam balance. An olefins plant based on autothermal cracking produces significantly less high-pressure steam than a conventional olefins plant, and so the use of an AAR system rather than a C₃ refrigeration system within an autothermal cracking process would have less of an impact on the process steam balance. Also, in addition to providing refrigeration to cool process streams, a C₃ refrigeration system in a conventional olefins plant recovers refrigeration value from cold process streams by warming them against the propylene refrigerant. Prior-art AAR systems were typically not designed in such a way that they could also recover refrigeration from cold process streams. Thus, these processes have not been useful in replacing the propylene refrigeration system in a conventional olefins manufacturing process.

AAR systems have been proposed for use in processes for the production of ethylene, as described in U.S. Pat. No. 4,143,521, issued to Pano et al. However, conventional cracking processes for the production of olefins, such as steam cracking furnaces, are generally operated at low pressure, and this limits the temperature of the liquid quench water that can be obtained. Typically, the maximum temperature of this water is in the range of about 80° C to about 99°C (approximately 180° F to 210°F). This relatively low-temperature water results in ammonia refrigerant being available at relatively warm temperatures for refrigeration, typically about 21 °C (70°F), and hinders the application of AAR in an olefins plant accordingly.

Use of AAR systems in ethylene plants was also suggested by D. Sohns and C. Fuge, "Reducing Consumption of Energy Is Possible in Olefin Plants," Oil & Gas Journal, September 13, 1976, pp 72-77. In this reference, the heat source to drive the AAR system was quench oil, a stream that is prone to fouling and is not available in all olefins plants. The authors state that the quench water stream in a conventional olefins plant is of little utility for providing refrigeration.

Although the temperature of liquid water obtainable can, in theory, be increased by operating the cracking process at higher pressures, this is not desirable for conventional furnace-based cracking processes because the optimum pressure for the furnace-based cracking reaction is generally less than about 2 bar. Thus, conventional furnace-based cracking processes typically use a C₃ refrigeration system, or a variation thereof to provide refrigeration at temperatures between ambient and about -45°C. C₃ refrigeration systems, as described in U.S. Pat. No 6,637,237 are generally powered by high pressure steam generated in the furnace-based cracking process. Although they are less energy efficient than AAR systems, this has not been a significant concern because conventional furnace-based cracking processes produce a large amount of high pressure steam which can be used for the C₃ refrigeration systems.

It would be highly desirable to make the ammonia refrigeration available at significantly lower temperatures, for example down to about -45°C (-50°F), while also recovering refrigeration value from the relatively low temperature process streams available in processes that produce olefins.

Surprisingly, we have now found that olefins may be advantageously produced by autothermal cracking of hydrocarbons at relatively high pressures, where the water from quenching of the autothermal cracking product stream is utilized to drive an AAR refrigeration system for purification of the product stream to produce said olefins. In particular, a beneficial feature of the present invention is that the ammonia refrigeration is made available at a lower temperature than the prior art processes, and that the AAR system can completely replace the conventional C₃ refrigeration system within olefins manufacturing plants based on the relatively high-pressure autothermal cracking of hydrocarbons.

### Summary of the Invention

One aspect of this invention is an autothermal cracking process for production and recovery of olefins. The process comprises feeding a substantially hydrocarbon feedstock and an oxygen-containing gas to an autothermal cracker to provide a hydrocarbon product stream comprising olefins. A waste enthalpy source generated by said autothermal cracking process is used to at least partially drive an ammonia absorption refrigeration system to provide chilling for at least one process stream in the separation and/or purification of olefins from the hydrocarbon product stream.

Another aspect of this invention is an ammonia absorption refrigeration process comprising at least one enthalpy source selected from the group consisting of: quench water generated through the cooling of cracked gases from an autothermal cracking reactor; steam generated through the cooling of cracked gases from an autothermal cracking reactor; sufficiently warm streams derived from processes which utilize the ethylene produced from the autothermal cracking process; and sufficiently warm streams from other chemical or refinery process units located near an autothermal cracking reactor.

### Brief Description of the Drawings

Figure 1 is a diagram of an autothermal cracking process for the manufacture of olefins.
Figure 2 is a diagram of an ammonia absorption refrigeration system incorporated into an autothermal cracking process
Figure 3 is an alternate diagram of an ammonia absorption refrigeration system incorporated into an autothermal cracking process

### Detailed Description of the Invention

This invention describes using an AAR system to recover olefins, including ethylene, from a cracked gas stream which is produced by an autothermal cracking reactor. There are many design options for the recovery of ethylene from a cracked gas stream. The process of the present invention may be used to convert both liquid and gaseous hydrocarbons into olefins. Suitable liquid hydrocarbons include naphtha, gas oils, vacuum gas oils and mixtures thereof. Preferably, however, gaseous hydrocarbons such as ethane, propane, butane and mixtures thereof are employed.

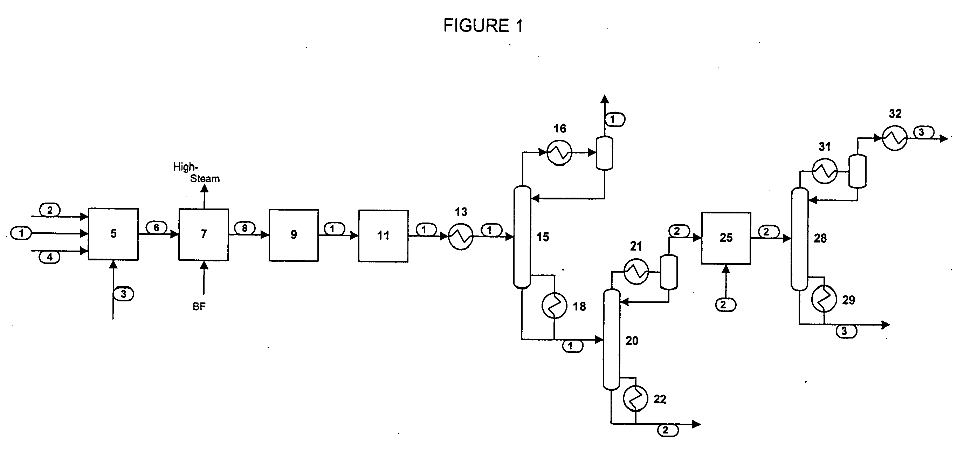

Figure 1 presents a general process for the production of ethylene via autothermal cracking. This process is described herein in order to better understand the use of an AAR system within such a process. Those skilled in the art will realize that many variations of the process configuration presented in Figure 1 can be conceived, particularly in the separation and purification section of the process. The specific configuration presented in Figure 1 does not limit the scope of the invention.

A substantially hydrocarbon feedstock to the process is shown a s stream 1. As used herein, the term "substantially hydrocarbon feedstock" refers to a hydrocarbon feedstock that generally comprises hydrocarbons consisting essentially of ethane, ethylene, propane, propylene, butane, butylene, diene and acetylenic compounds, and hydrocarbon impurities. It is combined with an oxygen-containing gas, shown as stream 2. Suitably, the oxygen-containing gas is molecular oxygen, air and/or mixtures thereof. The oxygen-containing gas may be mixed with an inert gas such as nitrogen or argon. Optionally, a recycle stream, shown as stream 3 and a hydrogen-containing stream, shown as stream 4 may enter the autothermal reactor 5. In the autothermal reactor 5, streams 1 through 4 can be preheated and are reacted to form a hot cracked gas, shown as stream 6. Preferably, the substantially hydrocarbon feedstock and oxygen-containing gas are fed to the autothermal reactor 5 at a ratio of hydrocarbon to oxygen-containing gas of about 5 to about 16 times, preferably about 5 to about 13.5 times, more preferably about 6 to about 10 times, the stoichiometric ratio of hydrocarbon to oxygen-containing gas required for complete combustion of the hydrocarbon to carbon dioxide and water.

The autothermal cracking catalyst is capable of supporting combustion beyond the fuel rich limit of flammability. The catalyst usually comprises a Group VIII metal as its catalytic component. Suitable Group VIII metals include platinum, palladium, ruthenium, rhodium, osmium and iridium. Rhodium, and more particularly, platinum and palladium are preferred. Typical Group VIII metal loadings range from about 0.01 to about 100 weight percent, preferably, between about 0.01 to about 20 weight percent, and more preferably, from about 0.01 to about 10 weight percent based on the total dry weight of the catalyst.

Where a Group VIII catalyst is utilized, it is preferably utilized in combination with a catalyst promoter. The promoter may be a Group IIIA, IVA, and/or VA metal. Alternatively, the promoter may be a transition metal; the transition metal promoter being a different metal to that which may be employed as the Group VIII transition metal catalytic component.

Preferred Group IIIA metals include AI, Ga, In and TI. Of these, Ga and In are preferred. Preferred Group IVA metals include Ge, Sn and Pb. Of these, Ge and Sn are preferred. The preferred Group VA metal is Sb. The atomic ratio of Group VIII B metal to the Group IIIA, IVA or VA metal may be about 1 : about 0.1 - 50.0, preferably, about 1: about 0.1 - 12.0.

Suitable metals in the transition metal series include those metals in Group IB to VIII of the Periodic Table. In particular, transition metals selected from Groups IB, IIB, VIB, VIIB and VIII of the Periodic Table are preferred. Examples of such metals include Cr, Mo, W, Fe, Ru, Os, Co, Rh, Ir, Ni, Pt, Cu, Ag, Au, Zn, Cd and Hg. Preferred transition metal promoters are Mo, Rh, Ru, Ir, Pt, Cu and Zn. The atomic ratio of Group VIII metal to transition metal promoter may b e about 1: about 0.1 - about 50.0, preferably, about 1: about 0.1 - about 12.0.

Preferably, the catalyst comprises only one promoter selected from Group IIIA, Group IVA, Group VB and the transition metal series. For example, the catalyst may comprise a metal selected from rhodium, platinum and palladium and a promoter selected from the group consisting of Ga, In, Sn, Ge, Ag, Au or Cu. Preferred examples of such catalysts include Pt/Ga, Pt/In, Pt/Sn, Pt/Ge, Pt/Cu, Pd/Sn, Pd/Ge, Pd/Cu and Rh/Sn. The Rh, Pt or Pd may comprise between about 0.01 and about 5.0 weight percent, preferably, between about 0.01 and about 2.0 weight percent, and more preferably, between about 0.05 and about 1.0 weight percent of the total weight of the catalyst. The atomic ratio of Rh, Pt or Pd to the Group IIIA, IVA or transition metal promoter may be about 1 : about 0.1 - about 50.0, preferably, about 1: about 0.1 - about 12.0. For example, atomic ratios of Rh, Pt or Pd to Sn may be about 1: 0.1 to about 50, preferably, about 1: 0.1 - about 12.0, more preferably, about 1: about 0.2 - about 3.0 and most preferably, about 1: about 0.5 - about 1.5. Atomic ratios of Pt or Pd to Ge, on the other hand, may be about 1: about 0.1 to about 50, preferably, about 1: about 0.1 - about 12.0, and more preferably, about 1: about 0.5 - about 8.0. Atomic ratios of Pt or Pd to Cu may be about 1: about 0.1 - about 3.0, preferably, about 1: about 0.2 - about 2.0, and more preferably, about 1: about 0.5 - about 1.5.

Alternatively, the promoter may comprise at least two metals selected from Group IIIA, Group IVA and the transition metal series. For example, where the catalyst comprises platinum, the platinum may be promoted with two metals from the transition metal series, for example, palladium and copper. Such Pt/Pd/Cu catalysts may comprise palladium in an amount of about 0.01 to about 5 weight percent, preferably, about 0.01 to about 2 weight percent, and more preferably, about 0.01 to about 1 weight percent based on the total weight of the dry catalyst. The atomic ratio of Pt to Pd may be about 1: about 0.1 - about 10.0, preferably, about 1: about 0.5 - about 8.0, and more preferably, about 1: about 1.0 - about 5.0. The atomic ratio of platinum to copper is preferably about 1 : about 0.1 - about 3.0, more preferably, about 1: about 0.2 - about 2.0, and most preferably, about 1: about 0.5 - about 1.5. Where the catalyst comprises platinum, it may alternatively be promoted with one transition metal, and another metal selected from Group IIIA or Group IVA of the periodic table. In such catalysts, palladium may be present in an amount of about 0.01 to about 5 weight percent, preferably, about 0.01 to about 2.0 weight percent, and more preferably, about 0.05 - about 1.0 weight percent based on the total weight of the catalyst. The atomic ratio of Pt to Pd may be about 1: about 0.1 - about 10.0, preferably, about 1: about 0.5 - about 8.0, and more preferably, about 1: about 1.0 - about 5.0. The atomic ratio of Pt to the Group IIIA or IVA metal may be a bout 1: about 0.1 - about 60, preferably, about 1: about 0.1 - about 50.0. Preferably, the Group IIIA or IVA metal is Sn or Ge, most preferably, Sn.

For the avoidance of doubt, the Group VIII metal and promoter in the catalyst may be present in any form, such as a metal, or in the form of a metal compound, such as an oxide.

The catalyst may be unsupported, such as in the form of a metal gauze, but is preferably supported. Any suitable support may be used such as ceramic or metal supports, but ceramic supports are generally preferred. Where ceramic supports are used, the composition of the ceramic support may be any oxide or combination of oxides that is stable at high temperatures of, for example, between about 600°C and about 1200°C. The support material preferably has a low thermal expansion coefficient, and is resistant to phase separation at high temperatures.

Suitable ceramic supports include corderite, lithium aluminum silicate (LAS), alumina (α-Al₂O₃), yttria stabilized zirconia, alumina titanate, niascon, and calcium zirconyl phosphate. The ceramic supports may be wash-coated, for example, with γ-Al₂O₃ .

The catalyst capable of supporting combustion beyond the fuel rich limit of flammability may be prepared by any method known in the art. For example, gel methods and wet-impregnation techniques may be employed. Typically, the support is impregnated with one or more solutions comprising the metals, dried and then calcined in air. The support may be impregnated in one or more steps. Preferably, multiple impregnation steps are employed. The support is preferably dried and calcined between each impregnation, and then subjected to a final calcination, preferably, in air. The calcined support may then be reduced, for example, by heat treatment in a hydrogen atmosphere.

The hydrocarbon-containing feedstock is passed over the autothermal cracking catalyst at a gas hourly space velocity of greater than about 10,000 h ⁻¹, preferably above about 20,000 h ⁻¹ and most preferably, greater than about 100,000 h ⁻¹. It will be understood, however, that the optimum gas hourly space velocity will depend upon the pressure and nature of the feed composition.

Additional feed components may be co-fed into the autothermal cracking reactor 5, such as hydrogen, carbon monoxide, carbon dioxide or steam. Preferably, the reactant mixture of hydrogen co-fed with the hydrocarbon-containing feedstock and oxygen-containing gas into the autothermal cracking reactor 5, and preheated prior to contact with the catalyst. Suitably, the molar ratio of hydrogen to oxygen-containing gas is in the range about 0.2 to about 4. Hydrogen co-feeds are advantageous because, in the presence of the catalyst, the hydrogen combusts preferentially relative to the hydrocarbon, thereby increasing the olefin selectivity of the overall process. Generally, the reactant mixture is preheated to temperatures below the auto ignition temperature of the reactant mixture.

A heat exchanger may be employed to preheat the reactant mixture prior to contact with the catalyst. The use of a heat exchanger may allow the reactant mixture to be heated to high preheat temperatures such as temperatures at or above the autoignition temperature of the reactant mixture. The use of high pre-heat temperatures is beneficial in that less oxygen reactant is required which leads to economic savings. Additionally, the use of high preheat temperatures can result in improved selectivity to olefin product. It has also been found that the use of high preheat temperatures enhances the stability of the reaction within the catalyst thereby leading to higher sustainable superficial feed velocities, and also reduces the thermal gradient experienced across the catalyst.

The autothermal cracking process may suitably be carried out at a catalyst exit temperature in the range of about 600°C to about 1200°C, preferably, in the range of about 850°C to about 1050°C and, most preferably, in the range of about 900°C to about 1000°C. To avoid further reactions taking place, the product stream is preferably is cooled to between about 600°C and about 750°C within 20 milliseconds of formation to form the hot cracked gas stream 6. Advantageously, if the autothermal cracking process is operated at a pressure of greater than about 20 barg, the products are cooled to between about 600°C and about 750°C within 10 milliseconds of formation. The autothermal cracking of the present invention is operated at a pressure of greater than about 5 barg. Preferably the autothermal cracking process is operated at a pressure of between about 5 to about 40 barg and preferably between about 10 to about 30 barg.

The hot cracked gas, shown as stream 6 typically contains ethylene, methane, hydrogen, carbon monoxide, carbon dioxide, ethane, and hydrocarbons heavier than ethane. The hot cracked gas stream 6 is cooled to by indirect heat exchange with boiler feed water in the primary cooling system, shown as step 7. This system generally has one or more heat exchangers and produces high-pressure steam and a cooled cracked gas, shown as stream 8.

The cooled cracked gas stream 8 is further cooled in a quench section, shown as step 9. The cooling processes employed, which are well known to those skilled in the art of ethylene manufacture, typically involve at least a contacting vessel in which the direct contact of the cracked gas with a circulating cooled water stream takes place. An additional step of direct contact with a circulating cooled hydrocarbon stream may optionally be employed within the quench section 9. The contacting operation generates a cooled cracked gas, shown as stream 10 and a warmed quench water stream. This warmed quench water is typically cooled in one or more exchangers and re-circulated to the direct contact vessel (the quench water circuit is not shown in Figure 1).

Stream 10 can be directed into a compression and contaminant removal step 11. Within this step the cooled cracked gas stream 10 is compressed to a pressure suitable for the downstream separation section, and contaminants are removed. For example, an amine or caustic scrubber may be employed to remove carbon dioxide and other acid gases from the cracked gas. Water is also typically removed from the cracked g as by condensation and/or adsorbent driers. If the autothermal cracking reaction is carried out at a sufficiently high pressure, the cracked gas may not need compression within step 11. In this case only contaminant removal would take place within step 11.

The high-pressure, essentially contaminant free cracked gas, shown as stream 12 is then chilled and partially condensed in exchanger 13. In practice, exchanger 13 would typically represent a series of exchangers and vapor/liquid separation vessels in which the cracked gas is progressively cooled and partially condensed by various cold process streams and progressively colder levels of external refrigeration. In a typical olefins plant this refrigeration would be supplied by a propylene refrigeration system and an ethylene or mixed refrigerant refrigeration system. The chilled cracked gas, shown as stream 14, typically enters the demethanizer column 15 at a temperature less than about -35°C. For simplicity stream 14 is depicted in Figure 1 as a single stream. In practice it would consists of a number of distinct vapor and liquid streams from the various chilling/partial condensation and vapor/liquid separation steps.

The demethanizer column 15 separates the methane and lighter components from the ethylene and heavier components in the cracked gas. The column is refluxed using partial condenser 16. The net overhead product of the demethanizer, stream 17, contains primarily methane, hydrogen, and carbon monoxide and little, if any, ethylene and heavier components. Stream 17 can be directed to hydrogen and/or CO recovery sections if desired, or used as fuel. The demethanizer 15 is reboiled with exchanger 18. The bottoms product of the demethanizer, stream 19, contains primarily ethylene and heavier components and little, if any, methane and lighter components.

Stream 19 enters the deethanizer column 20, which separates the ethane and lighter components from those heavier than ethane. The deethanizer 20 is refluxed with condenser 21 and reboiled with exchanger 22. The bottoms product, shown as stream 23, contains primarily hydrocarbons heavier than ethane. Stream 23 can be further treated to recover one or m ore of the heavy hydrocarbons i f desired. The deethanizer net overhead product, shown as stream 24, enters an acetylene conversion system, shown as step 25. This system typically contains a number of exchangers and reactors arranged such that the deethanizer overhead stream 24 is first heated, then essentially all of the acetylene in the stream is reacted with an external hydrogen stream 26, whereupon the stream is cooled again.

The essentially acetylene free, cooled stream 27 enters a C₂ splitter column 28, which purifies the ethylene sufficiently to be sold as a commercial product. The C₂ splitter is refluxed with condenser 31 and reboiled with exchanger 29. The n et overhead product is typically reheated in exchanger 32 and then withdrawn as the final purified ethylene product, shown as stream 33. As is weft-known by those skilled in the art, a pasteurizing section can be utilized on the top section of the C₂ splitter column 2 8. In this case the final ethylene product would be withdrawn a s a liquid from an intermediate stage of column 28, and the top vapor stream would serve as a vent for light gases. The bottoms product 30 contains primarily ethane and can be recycled to the reactor section or sold as a product.

It has been discovered that the autothermal cracking based olefins production process represented in Figure 1 exhibits surprising synergies with an AAR system. In particular, there is a waste enthalpy source in the form of quench water from step 9, and a need for refrigeration in the range from ambient temperature to about -45°C in, for example, exchanger series 13 and condensers 21 and 31. As used herein, a "waste enthalpy source" can be considered to be any suitable source of heat energy which would otherwise be rejected to the environment at or near ambient temperature, cannot be otherwise economically used within the autothermal cracking process or any nearby processes, or cannot be economically transformed into a different useful form of energy. For example, the majority of the heat energy within the quench water stream would be rejected to cooling water or another ambient cooling medium such as air. In this context, a "waste enthalpy source" would also include steam which is generated by the autothermal cracking unit which is in excess of what is needed as energy input to the autothermal cracking process or any nearby processes, and which cannot be economically transformed into a different useful form of energy such as electricity or mechanical power.

This synergy is exhibited when the autothermal cracking reactor 5 and therefore the quench step 9 are operated at a relatively high pressure, above about 5 bar absolute. In this case the quench water from step 9 can be recovered at a sufficiently high temperature to efficiently contribute to the operation of an AAR system.

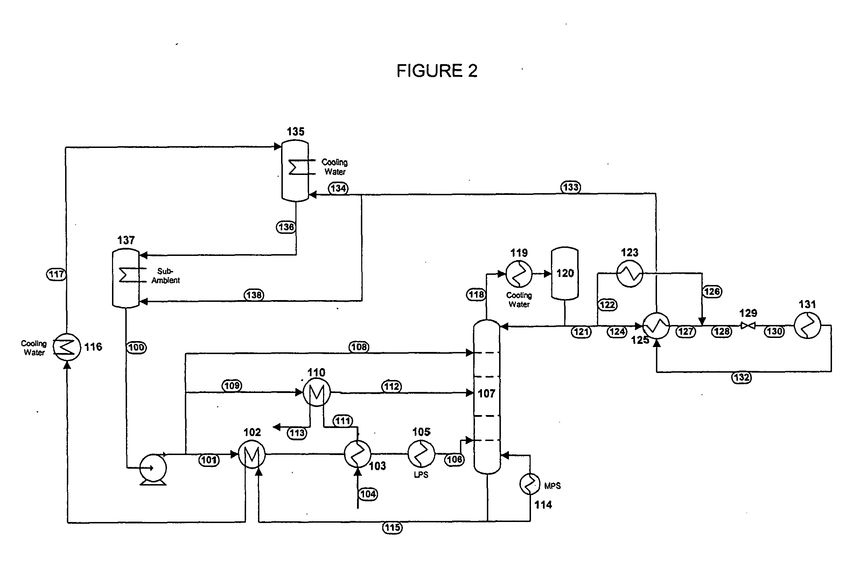

Figure 2 depicts one embodiment this invention wherein a waste enthalpy source, in this case heat from the quench water of step 9, is used to at least partially drive an AAR system, which is itself utilized in the separation and/or purification of olefins from the cracked gas stream. As used herein, "at least partially drive" means that the heat from the waste enthalpy source is used to pre-heat at least a portion of the rich ammonia aqua stream prior to entering the ammonia generator column 107 and/or to provide heat to reboil the ammonia generator column itself. Stream 100 is a rich ammonia aqua stream. It is pumped to around 15 bar and split into two streams. One portion, stream 101, is heated in exchanger 102 and then further heated in exchanger 103 against a quench water stream 104 from step 9 of Figure 1. The temperature of this quench water stream is at least a bout 95 C, and preferably at least about 110 C. The rich ammonia aqua stream can optionally be further heated against low-pressure steam (typically 2-10 bar) in exchanger 105 after which it enters as stream 106 at a relatively low location on the ammonia generator column 107.

A second portion of stream 100 enters the ammonia generator column 107 at a relatively high location a s stream 108. Optionally, a third portion of stream 100, stream 109, can be heated in exchanger 110 against the partially cooled quench water stream 111. The resulting heated stream 112 enters the ammonia generator column 107 at a middle location. At least a part of the cooled quench water stream 113 can be returned directly to step 9 or it can be further cooled before returning to step 9.

Many other types of arrangements for heating the rich ammonia aqua stream 100 and feeding it to the ammonia generator column can be envisioned by those skilled in the art of process design and optimization. The optimal column feed design will depend on the available utilities as well as economic (e.g. capital cost) and operational factors. The invention includes all such design variations, including, but not limited to, multiple feed locations, multiple ammonia concentrations, and multiple levels of preheat.

The ammonia generator column 107 is reboiled with medium-pressure steam (typically 6-25 bar) in exchanger 114. Other high-temperature, preferably waste enthalpy sources, could be used to reboil column 107. The net bottom product stream 115 consists of relatively lean ammonia aqua (enriched in water relative to the rich ammonia aqua stream 100). It will typically consist of water with less than 50 weight percent ammonia, and preferably less than 20 weight percent ammonia. It is cooled in exchanger 102 by contact with the rich ammonia aqua stream 101, optionally further cooled against cooling water in exchanger 116, and then directed to the absorption section as stream 117.

The gross overhead product of the ammonia generator, stream 118, consists of essentially pure ammonia and is condensed in exchanger 119. This stream will typically consist of at least 95 weight percent ammonia, preferably at least 99 weight percent ammonia. At least a portion of this condensed ammonia is commonly vaporized by indirect transfer of heat from one or more streams within the autothermal cracking olefin production process to said condensed ammonia. Indirect heat transfer means that the refrigerant is not in direct contact with the material being cooled, but rather, the refrigerant and the material being cooled are on opposite sides of a heat transfer surface. The liquid ammonia product is sent to an ammonia accumulator 120. A portion of the liquid ammonia is returned to the top of the ammonia generator as reflux liquid. The remainder, the net liquid overhead product of the ammonia generator, is withdrawn at around 15 barg as stream 121. A portion of stream 121, stream 122 can be subcooled in exchanger 123 against a cold stream from the olefins separation process, for example a stream at a temperature below about 10°C. For example and with reference to Figure 1, subcooling in exchanger 123 could be provided by the reheating of the ethylene product stream in exchanger 32 or the reheating of light gases derived from stream 17. The remainder of stream 121, stream 124, is subcooled in exchanger 125 against returning cold ammonia vapor stream 132. The two subcooled streams 126 and 127 are combined into stream 128.

The pressure of stream 128 is reduced to a lower pressure, for example 0.5 to 1.0 barg through valve 129 or some other pressure-reducing means. The flashed and at least partially liquid stream 130 is directed to exchanger 131 where it is vaporized to provide refrigeration to as low as about -45°C to the olefins separation and purification process. Exchanger 131 will generally represent a number of individual exchangers to provide refrigeration to discrete points in the olefins separation and purification process. For example and with reference to Figure 1, this refrigeration can be directed to an exchanger within the chilling train represented by exchanger series 13, and to the condensers 21 and 31, among others.

The vaporized low-pressure ammonia stream 132 is reheated in exchanger 125. The resulting heated stream 133 is then split into two portions, stream 134 and 138. Stream 134 is directed to the ambient temperature absorber 135. In this absorber 135 the ammonia is absorbed into the lean ammonia aqua stream 117. The absorption of ammonia into water is exothermic. Thus, cooling water is used to cool the absorber to drive the absorption. The heat of absorption in absorber 135 is removed with cooling water or some other ambient-temperature cooling medium. The intermediate-concentration aqua stream 136 from absorber 135 is directed to the sub-ambient absorber 137 where it is contacted with the remainder of the vaporized ammonia, stream 138. The heat of absorption in absorber 137 is removed by a sub-ambient cooling medium. For example and with reference to Figure 1, the sub-ambient cooling medium could be provided by hydrocarbon vaporization in reboilers 18, 22, and/or 29. The ammonia is absorbed in absorbers 135 and 137, producing the rich ammonia aqua stream 100 from absorber 137.

Many other types of arrangements for absorbing the heated ammonia stream 133 into the lean ammonia aqua stream 117 can be envisioned by those skilled in the art. The optimal absorber system design will depend on the available ambient and sub-ambient cooling sources as well as economic (e.g. capital cost) and operational factors. The invention includes all such design variations, including, but not limited to, multiple absorption steps, absorption at multiple pressures, and multiple series/parallel arrangements of individual absorption steps

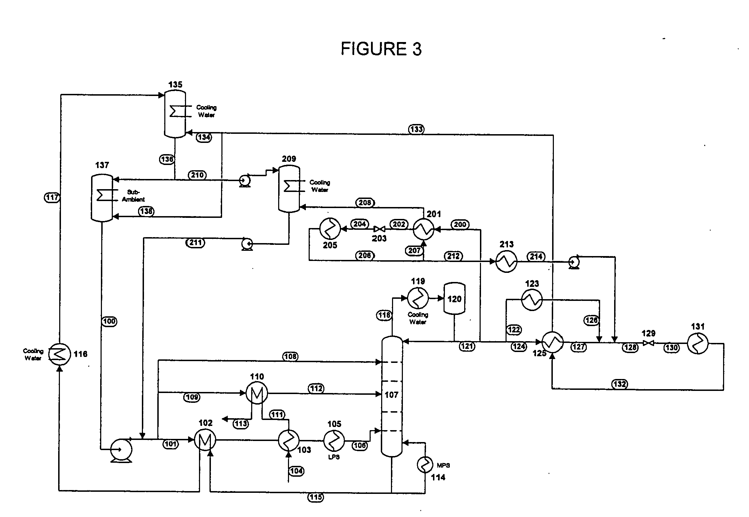

The embodiment of Figure 2 provides refrigeration at a single temperature level, in this case around -30°C to -45°C. It should be noted that the process of this invention is easily adapted to provide refrigeration at multiple temperature levels. Figure 3 depicts an arrangement of the process of this invention which provides refrigeration at two distinct temperatures. Many of the steps and streams in Figure 3 are identical to those in Figure 2 and therefore have identical number identifiers.

In the process of Figure 3, a portion of stream 121 is withdrawn as stream 200 and subcooled in exchanger 201 to produce subcooled liquid 202. This liquid is flashed across valve 203 to a pressure higher than that of stream 130, but lower than that of stream 202. For example, flashed stream 204 is at a pressure of 2 barg and is vaporized in refrigeration exchanger 205, providing refrigeration to the ethylene process at a temperature of about -9°C. The vaporized ammonia stream 206 is split into two streams. One portion, stream 207, is reheated in exchanger 201 and directed as stream 208 to the intermediate-pressure absorber 209. Here the ammonia is absorbed into stream 210, which is a portion of the intermediate-concentration aqua stream 136. A portion of the lean ammonia aqua stream 117 could also be used as the absorbent liquid. The heat of absorption in absorber 209 is removed by a suitable cooling medium, in this case cooling water.

The resulting rich ammonia aqua stream 211 is pumped and combined with the rich ammonia aqua stream 100 and subsequently fed to the ammonia generator column 107 as described above. Alternatively, the rich ammonia aqua stream 211 could be fed directly in one or more portions to the ammonia generator column 107. In general, depending on the design chosen, stream 211 could also be fed to the lower-pressure absorbers 135 and/or 137, combined with the rich aqua from absorbers 135 and/or 137, or fed separately to the ammonia generator column 107. All such variations are encompassed within the concept of this invention.

One aspect of this invention and its integration with an autothermal cracking process to produce olefins is that one or more sub-ambient temperature streams from the olefins recovery and purification process are heated in and thereby provide cooling duty to one or more parts of the ammonia absorption process of this invention. In each case the heating of these olefins related process streams improves the performance or efficiency of the AAR system of this invention. As described in Figure 2, liquid ammonia can be subcooled against one or more sub-ambient temperature streams from the olefins recovery and purification process in exchanger 123 to increase the amount of liquid and therefore the refrigeration duty available to the refrigeration exchangers 131. In this embodiment, the heat provided to the one or more process streams is derived from the subcooling of one or more liquid ammonia-containing streams to a sub-ambient temperature.

As further described in Figure 2, the absorption of ammonia is carried out under sub-ambient conditions in absorber 1 37 by cooling it with one or more sub-ambient temperature streams from the olefins recovery and purification process. Such sub-ambient ammonia absorption reduces the required circulation of lean ammonia aqua (stream 117), thereby reducing the energy required by reboiler 114 and therefore reducing the total energy used by the AAR system. In this embodiment, the heat provided to the one or more process streams is derived from the heat of solution arising from the absorption of an ammonia-containing vapor into an aqueous liquid at sub-ambient temperatures.

Figure 3 presents another embodiment for the recovery of refrigeration value from sub-ambient temperature streams from the olefins recovery and purification process to the AAR system. A portion of the intermediate-pressure vaporized ammonia stream 206 is directed as stream 212 to exchanger 213. There it is fully condensed by heat exchange with one or more sub-ambient temperature process streams from the ethylene recovery and purification process. The resulting liquid stream 214 can then be pumped as shown and combined with the subcooled streams 126 and 127 to form stream 128. As a result, additional liquid ammonia refrigerant can be generated without having to cycle through the relatively energy-intensive absorption/ammonia generation sequence. In this embodiment, the heat provided to the one or more process streams is derived from the at least partial condensation of one or more ammonia-containing vapor streams at a sub-ambient temperature.

In the present invention, streams within the olefins recovery and purification process are condensed and optionally desuperheated by exchange with ammonia refrigerant. The ammonia evaporation temperature is typically in the range of about 10°C to about -45°C. Ammonia is vaporized by heat indirectly transferred from the relevant steps discussed in Figures 1 through 3. Indirect heat transfer means that the refrigerant is not in direct contact with the material being cooled, but rather, the refrigerant and the material being cooled are on opposite sides of a heat transfer surface.

In a typical olefins recovery and purification process refrigeration is required at temperatures below that which can be provided by a C₃ or AAR refrigeration system. In practice, refrigeration at these colder temperatures is typically provided by a separate ethylene refrigeration system or a mixed refrigeration system. Ammonia evaporation temperatures as low as about -45°C can readily be achieved in an AAR system comprising a n ammonia refrigerant. This is sufficient to condense ethylene refrigerant at typical ethylene refrigeration compressor discharge pressures. It is also sufficient to provide condensing duty to a mixed refrigeration stream. Thus, the combination of AAR and an ethylene refrigeration system, or AAR and a mixed refrigeration system is sufficient to provide all of the net refrigeration needs within the ethylene recovery and purification process.

Advanced AAR cycles, including multi-stage absorption refrigeration systems, multiple-lift refrigeration cycles, advanced absorption vapor exchange GAX cycles, and multiple effect absorption cycles, as described in U.S. Pat. No. 5,097,676, U.S.Pat. No. 5,966,948, Erickson and Tang, "Evaluation of Double-Lift Cycles for Waste Heat Powered Refrigeration," Intl. Absorption Conf., Montreal, Canada, Sept. 17-22 (1996), Erickson, Potnis, and Tang, "Triple Effect Absorption Cycles," Proc. Intersoc. Energy Convers. Eng. Conf. (1996), 31^{st}, 1072-1077, Rane and Erickson, "Advanced absorption cycle: vapor exchange GAX," Am. Soc. Mech. Eng. (1994) 25-32, and Richter, "Multi-Stage Absorption Refrigeration Systems," Journal of Refrigeration, September/October 1962, are hereby incorporated by reference. Advanced AAR cycles can use less heat and lower temperature heat sources while providing refrigeration at lower temperatures than simpler AAR processes. Furthermore, the advanced AAR cycles can accommodate refrigeration at multiple temperature levels and heat sources at multiple levels. Advanced AAR cycles can have multiple absorbers and multiple ammonia fractionators.

It is generally most preferred to completely forego or replace the propane or propylene refrigeration circuit with an AAR circuit, since this allows complete elimination of the energy intensive C₃ compressor, condenser, flash drums, and other equipment associated with the circuit, as well as elimination of the utilities consumption associated with running the C₃ compressor. The evaporators are generally the interface between the refrigeration circuit and the process. The process stream being cooled is on the hot side of the evaporator, and evaporating refrigerant is on the cold side of the evaporator. Thus, when using an AAR system to replace a C₃ refrigeration circuit, the evaporators retain their function and boiling ammonia refrigerant replaces boiling C₃ refrigerant on cold side of the evaporator.

Replacing the C₃ refrigeration circuit of an autothermal cracking process with an AAR system will generally result in lower energy consumption and higher waste heat utilization. The major power input to conventional C₃ refrigeration cycles is in the form of electricity or high-pressure steam used to power the compressor motor. The major power to an AAR unit is the waste enthalpy source used to preheat feeds to the ammonia fractionator and/or to reboil the ammonia fractionator. The waste enthalpy source is essentially free energy, since it is otherwise lost to the environment via air or water cooling. Thus, replacing the C₃ refrigeration cycle with an AAR refrigeration cycle generally leads to savings of at least the electricity or steam required to power the drivers of the propane or propylene compressors, since only a small amount of electricity or steam is required to power the drivers of the pumps associated with the AAR.

The use of an AAR system allows the elimination of the conventional C₃ system, and provides a more energy efficient refrigeration system. Thus, the AAR system may be used to provide all net refrigeration duty between about -45°C and ambient temperature for the autothermal cracking olefins plant. In addition, the use of an AAR system utilizes the waste enthalpy source (from the quench water) that would otherwise be lost and the use of an AAR system reduces the high pressure steam requirement for the overall olefins plant. This is particularly beneficial for autothermal cracking processes since such processes produce significantly less high pressure steam than conventional furnace-based crackers.

For this invention, it is preferable that at I east portion of the waste enthalpy source used in the AAR fractionator is derived from a heat source available from the autothermal cracking process, from a unit that produces feed for the autothermal cracking process, or from a unit that is located near the autothermal cracking process. Suitable sources of heat to the AAR ammonia fractionator are those that are available at a supply temperature of at least 95°C, and preferably at least 110°C for best results. Higher waste enthalpy source stream temperatures are preferred since they generally lead to higher AAR process efficiency.

One suitable waste enthalpy source on the autothermal cracking unit is the quench water generated through the cooling of cracked gases from an autothermal cracking reactor.

Another suitable waste enthalpy source could be saturated high-pressure steam generated through the cooling of cracked gases from an autothermal cracking reactor, or waste low- or medium-pressure steam from the autothermal cracking process.

Still another suitable waste enthalpy source may be derived from processes which utilize the ethylene produced from the autothermal cracking process, such as polyethylene or ethylene oxide manufacture.

Waste enthalpy sources for the AAR are not limited to those described. They can also include heat sources available on other nearby chemical or refinery process units, and steam which may be available from these units or site utilities units. The use of a waste heat enthalpy source from autothermal cracking process streams, heat from processes that produce a feed stream for the autothermal cracking process, or heat sources available on other chemical or refinery process units located near the a utothermal cracking process provides synergy between the a utothermal cracking process and these other processes.

The process of the present invention results in substantial benefits over alternative autothermal cracking processes. One benefit is that utilizing AAR for autothermal cracking processes in accordance with the present invention allows for the elimination of a propane or propylene refrigeration loop commonly used in conventional autothermal cracking processes. This eliminates the expensive C₃ compressor, condenser, flash drums, and other equipment associated with the circuit, as well as elimination of the capitalized utilities associated with running the C₃ compressor. The cost of suitable AAR in accordance with the present invention is substantially lower than conventional propane or propylene vapor recompression systems.

Another benefit of utilizing AAR within an autothermal cracking process is that replacing the C₃ refrigeration cycle will lead to energy savings approximately equal to the electricity or steam required to power the drivers of the propane or propylene compressors of a conventional autothermal cracking process, since a relatively smaller amount of electricity and/or steam is required to power the drivers of the pumps associated with AAR system and to provide other process heat required by the AAR system.

Another benefit is that utilizing AAR within an autothermal cracking process in accordance with the present invention consumes waste heat enthalpy from the autothermal cracking process for preheating the feed to the ammonia fractionator. Waste enthalpy sources are essentially a free enthalpy source, since it is otherwise lost to the environment via air or water cooling.

Another benefit is that the AAR system is driven by pumps for conveying liquids as compared to refrigeration compressors for conveying gas. Refrigeration compressors are far more costly and require more energy to operate than pumps that convey liquid. Since compression often results in an elevation in the temperature of the- compressed gas due to compressor inefficiency, inevitably, additionally cooling utilities are required and energy lost.

Another benefit is that utilizing AAR for autothermal cracking processes in accordance with the present invention also reduces greenhouse gas emissions. The use of waste heat powered AAR in autothermal cracking processes generally results in a substantial reduction in electricity or high-pressure steam consumption from the overall replacement of vapor recompression refrigeration compressors with AAR pumps. Reducing electricity or high-pressure steam consumption generally leads to lower CO₂ emissions, since incremental electricity or high-pressure steam most often derives from fossil fuel fired power plants or plant furnaces.

The process has been described for the purposes of illustration only in connection with certain embodiments. However, it is recognized that various changes, additions, improvements, and modifications to the illustrated embodiments may be made by those persons skilled in the art, all falling within the scope and spirit of the invention.

### Example 1

This example describes the process of the present invention for recovering olefins, and in particular ethylene, from a mixed hydrocarbon stream derived from the effluent of an autothermal cracking reactor. The ammonia absorption process of this example was simulated using a commercially available process simulation package. The process simulated in the example is identical to the embodiment of Figure 2, except that exchangers 105, 110 and 116 are not used, and stream 109 has zero flow. Selected stream information is given in Table 1, with stream numbers referenced to Figure 2. Exchanger and absorber duties for the example (in MW) are given in Table 2.

Exchanger 131, the net refrigeration duty supplied by the ammonia absorption refrigeration system of this invention, is depicted in Figure 2 as a single exchanger. In this example there are three separate refrigeration exchangers employed, corresponding to the C₂ splitter condenser, cracked gas chilling, and low-temperature refrigeration condensing duties in the ethylene recovery and purification process. The duty for exchanger 131 shown in Table 2 is the sum of these three exchangers. Cooling of the ammonia stream 122 in exchanger 123 is provided by the reheating of cold fuel gases from the ethylene recovery process, and the sub-ambient cooling in absorber 137 is provided by a combination of the deethanizer reboiler and a portion of the C2 splitter reboiler duties in the ethylene recovery and purification process. Column 107 is reboiled using medium-pressure (13 bar) steam and condensed against cooling water.

**Table 1 Flows and Conditions for Streams of Example 1**

| Stream | Temperature Deg C | Pressure Barg | Vapor Fraction | Molar Flow (kg mol/hr) | |
|---|---|---|---|---|---|
| | | | | WATER | AMMONIA |
| 100 | 18.9 | -0.50 | 0.000 | 45148.1 | 15417.6 |
| 101 | 19.1 | 15.99 | 0.000 | 38889.0 | 13280.2 |
| 106 | 148.6 | 15.99 | 0.077 | 38889.0 | 13280.2 |
| 108 | 19.1 | 15.99 | 0.000 | 6254.6 | 2135.9 |
| 115 | 172.1 | 15.14 | 0.000 | 45147.1 | 5306.4 |
| 117 | 25.0 | 15.14 | 0.000 | 45147.1 | 5306.4 |
| 118 | 44.2 | 15.02 | 1.000 | 1.4 | 15115.1 |
| 121 | 41.5 | 15.00 | 0.000 | 1.0 | 10111.2 |
| 122 | 41.5 | 15.00 | 0.000 | 0.5 | 5561.1 |
| 124 | 41.5 | 15.00 | 0.000 | 0.4 | 4550.0 |
| 128 | -12.6 | 14.60 | 0.000 | 1.0 | 10111.2 |
| 132 | -42.9 | -0.40 | 0.990 | 1.0 | 10111.2 |
| 133 | 38.7 | -0.50 | 1.000 | 1.0 | 10111.2 |
| 134 | 38.7 | -0.50 | 1.000 | 0.6 | 6168.1 |
| 136 | 30.0 | -0.50 | 0.000 | 45147.7 | 11474.5 |
| 138 | 38.7 | -0.50 | 1.000 | 0.4 | 3943.1 |

If the high-temperature quench water stream 104 were not u sed to pre-heat the rich ammonia-water solution in exchanger 103, an additional 12 MW of steam thermal energy would be required in the ammonia generator, either as additional medium-pressure steam in reboiler 114, or as low-pressure (5 bar) steam in exchanger 105. This corresponds to about 20,500 kg/hr of medium- or low-pressure steam. Therefore the use of waste heat in the quench water allows for a significant savings in higher-value steam energy.

**Table 2 Heat Exchanger Duties for Example 1**

| Exchanger | Net Duty (MW) |
|---|---|
| 102 | 161.46 |
| 103 | 38.69 |
| 105 | Not Used |
| 110 | Not Used |
| 116 | Not Used |
| 114 | 83.80 |
| 119 | -78.60 |
| 123 | -5.02 |
| 125 | 8.76 |
| 131 | 59.16 |
| 135 | -51.83 |
| 137 | -46.94 |

### Example 2

This example describes the process of the present invention for recovering olefins, and in particular ethylene, from a mixed hydrocarbon stream derived from the effluent of an autothermal cracking reactor. In this example both low-temperature and intermediate-temperature ammonia refrigeration circuits are used, and there is direct recuperation of ammonia refrigerant in exchanger 213. The process simulated in this example is identical to the preferred embodiment of Figure 3, except that exchangers 105, 110 and 116 are again not used, and stream 109 has zero flow. Selected stream information for this example is given in Table 3, with stream numbers referenced to Figure 3. Exchanger and absorber duties for the example (in MW) are given in Table 4.

As in Example 1, exchanger 131 is depicted in Figure 3 as a single exchanger, while in this example it represents three separate refrigeration exchangers, corresponding to the C₂ splitter condenser, cracked gas chilling, and low-temperature refrigeration condensing duties in the olefins recovery and purification process. The duty for exchanger 131 shown in Table 4 is the sum of these three exchangers. The intermediate-temperature refrigeration in exchanger 205 is delivered a t about -9°C, and recuperation of a portion of the ammonia vapor generated in exchanger 205 is carried out in exchanger 213. The intermediate-pressure absorber 209 is cooled with cooling water. The cooling and heating media in the other exchangers and absorbers are similar to those described in Example 1.

This example demonstrates the flexibility of the process of this invention to providing refrigeration at a number of temperatures, and the ability to recuperate refrigeration using cold process streams, for example in exchangers 123 and 213 and absorber 137.

In this example, if the high-temperature quench water stream 104 were not used to pre-heat the rich ammonia-water solution in exchanger 103, an additional 15-16 MW of steam thermal energy would be required in the ammonia generator, either as additional medium-pressure steam in reboiler 114, or as low-pressure (5 bar) steam in exchanger 105. This corresponds to about 26,000 - 27,000 kg/hr of medium- or low-pressure steam. Therefore the use of waste heat in the quench water allows for a significant savings in higher-value steam energy.

**Table 3 Flows and Conditions for Streams of Example 2**

| Stream No. | Temperature (Deg C) | Pressure (barg) | Vapor Fraction | Molar Flow (kg mol/hr) | |
|---|---|---|---|---|---|
| | | | | WATER | AMMONIA |
| 100 | 17.7 | -0.50 | 0.000 | 42645.7 | 14752.5 |
| 101 | 19.3 | 15.99 | 0.000 | 38341.2 | 14041.6 |
| 106 | 148.6 | 15.99 | 0.101 | 38341.2 | 14041.6 |
| 108 | 19.3 | 15.99 | 0.000 | 6145.9 | 2250.8 |
| 115 | 172.1 | 15.14 | 0.000 | 44490.5 | 5229.3 |
| 117 | 25.0 | 15.14 | 0.000 | 44490.5 | 5229.2 |
| 118 | 44.2 | 15.02 | 1.000 | 1.6 | 16662.4 |
| 121 | 41.5 | 15.00 | 0.000 | 1.0 | 11064.7 |
| 122 | 41.5 | 15.00 | 0.000 | 0.5 | 5103.3 |
| 124 | 41.5 | 15.00 | 0.000 | 0.4 | 4175.4 |
| 128 | -15.6 | 14.60 | 0.000 | 0.9 | 9993.1 |
| 132 | -42.9 | -0.40 | 0.990 | 0.9 | 9993.1 |
| 133 | 35.9 | -0.50 | 1.000 | 0.9 | 9993.1 |
| 134 | 35.9 | -0.50 | 1.000 | 0.6 | 6096.1 |
| 136 | 30.0 | -0.50 | 0.000 | 44491.1 | 11325.3 |
| 138 | 35.9 | -0.50 | 1.000 | 0.4 | 3897.1 |
| 200 | 41.5 | 15.00 | 0.000 | 0.2 | 1786.0 |
| 202 | 29.3 | 14.60 | 0.000 | 0.2 | 1786.0 |
| 206 | -8.9 | 2.00 | 0.990 | 0.2 | 1786.0 |
| 207 | -8.9 | 2.00 | 0.990 | 0.1 | 1071.6 |
| 208 | 38.7 | 1.90 | 1.000 | 0.1 | 1071.6 |
| 210 | 30.0 | -0.50 | 0.000 | 1845.8 | 469.8 |
| 212 | -8.9 | 2.00 | 0.990 | 0.1 | 714.4 |
| 214 | -9.9 | 1.90 | 0.000 | 0.1 | 714.4 |

**Table 4 Heat Exchanger Duties for Example 2**

| Exchanger | Net Duty (MW) |
|---|---|
| 102 | 159.15 |
| 103 | 50.50 |
| 105 | Not Used |
| 110 | Not Used |
| 116 | Not Used |
| 114 | 82.74 |
| 119 | -86.63 |
| 123 | -5.02 |
| 125 | 8.37 |
| 131 | 59.16 |
| 135 | -51.13 |
| 137 | -46.94 |
| 201 | 0.58 |
| 205 | 9.13 |
| 209 | -8.12 |
| 213 | -4.35 |

## Claims

1. An autothermal cracking process for the production and recovery of olefins, wherein said process comprises:
(a) feeding a substantially hydrocarbon feedstock and an oxygen-containing gas to an autothermal cracker to provide a hydrocarbon product stream comprising olefins,
(b) utilizing a waste enthalpy source generated by said autothermal cracking process to at least partially drive an ammonia absorption refrigeration system to provide chilling for at least one process stream in the separation and/or purification of olefins from the hydrocarbon product stream.

2. The process of Claim 1 wherein said waste enthalpy source is provided at a temperature of at least about 95°C.

3. The process of Claim 1 wherein said waste enthalpy source is provided at a temperature of at least about 110°C.

4. The process of Claim 1 wherein said waste enthalpy source is provided in the form of a quench water stream.

5. The process of Claim 1 wherein said waste enthalpy source is provided in the form of steam.

6. The process of Claim 1 wherein said waste enthalpy source is provided in form of a combination of steam and a quench water stream.

7. The process of Claim 1 wherein said substantially hydrocarbon feedstock comprises hydrocarbons consisting essentially of ethane, ethylene, propane, propylene, butane, butenes, diene and acetylenic compounds, and hydrocarbon impurities.

8. The process of Claim 1 wherein said separation and purification steps in part (b) results in the recovery of ethylene.

9. The process of Claim 1 wherein step (b) provides chilling at a temperature lower than 10 °C.

10. The process of Claim 9 wherein s aid chilling is provided for separation and purification steps comprising (i) chilling and partial condensation of the hydrocarbon product stream; (ii) providing chilling to generate reflux liquid for one or more distillation columns; (iii) providing chilling to at least partially condense the working fluid of a lower-temperature refrigeration system.

11. The process of Claim 1 wherein said autothermal cracker of step (a) is operated at a pressure range of between about 5 barg and about 40 barg.

12. The process of Claim 11 wherein said autothermal cracker of step (a) is operated at a pressure range of between about 20 barg and about 30 barg.

13. The process of Claim 1, wherein the hydrocarbon-containing feedstock and oxygen-containing gas are fed to the autothermal cracker at a ratio of hydrocarbon to oxygen-containing gas of about 5 to about 16 times the stoichiometric ratio of hydrocarbon to oxygen-containing gas required for complete combustion of the hydrocarbon to carbon dioxide and water.

14. The process according to claim 1, wherein hydrogen is co-fed with the hydrocarbon-containing feedstock and oxygen-containing gas into the autothermal cracker.

15. The process according to claim 14, wherein the molar ratio of hydrogen to oxygen-containing gas is in the range about 0.2 to about 4.

16. The process according to claim 1, wherein the AAR system may also be utilized to provide heat to one or more process streams which are available at sub-ambient temperatures.

17. The process according to claim 16 wherein the heat provided to one or more process streams is derived from the heat of solution arising from the absorption of an ammonia-containing vapor into an aqueous liquid at sub-ambient temperatures.

18. The process according to claim 16 wherein the heat provided to one or more process streams is derived from the subcooling of one or more liquid ammonia-containing streams to a sub-ambient temperature.

19. The process according to claim 16 wherein the heat provided to one or more process streams is derived from the at least partial condensation of one or more ammonia-containing vapor streams at a sub-ambient temperature.

20. An ammonia absorption refrigeration process comprising at least one enthalpy source selected from the group consisting of: quench water generated through the cooling of cracked gases from an autothermal cracking reactor; steam generated through the cooling of cracked gases from an autothermal cracking reactor; sufficiently warm streams derived from processes which utilize the ethylene produced from the autothermal cracking process; and sufficiently warm streams on other chemical or refinery process units located near an autothermal cracking reactor,

21. The process of Claim 20 wherein said enthalpy source has a temperature of at least about 95°C.

22. The process of Claim 20 wherein said enthalpy source has a temperature of at least about 110°C.

23. The process of Claim 20 wherein said enthalpy source is used to provide heat to an ammonia generator column.

24. The process of claim 23 wherein said enthalpy source is used to provide heat to one or more feeds entering said ammonia generator column.
